# EUROPEAN PATENT APPLICATION

(11) **EP 2 927 319 A1**
(43) Date of publication of application: **07.10.2015**
(21) Application number: 14162678.8
(22) Date of filing: 31.03.2014
(51) Int. Cl.: C12N 9/96, C12N 11/06, C12N 9/04, G01N 33/66

(54) **High load enzyme immobilization by crosslinking**

(71) Applicant: Roche Diagnostics GmbH, 68305 Mannheim (DE); F. Hoffmann-La Roche AG, 4070 Basel (CH)
(72) Inventor: The designation of the inventor has not yet been filed
(74) Representative: Herzog, Fiesser & Partner Patentanwälte PartG mbB

(57) **Abstract**

The present invention relates to a method for cross-linking polypeptide molecules, comprising the step of combining a cross-linking agent and polypeptide molecules in solution under conditions suitable for a cross-linking reaction to occur and to a preparation comprising cross-linked polypeptide molecules obtainable by said method. The present invention further relates to cross-linked polypeptide molecules, wherein said polypeptide molecules are cross-linked by essentially unbranched cross-linking groups comprising at least 40 contiguous atoms. Moreover, the present invention relates to a test chemistry matrix comprising a redox cofactor, an agent capable of eliciting a change in at least one measurable property of an indicator reagent in the presence of redox equivalents, and an indicator reagent, wherein said chemistry matrix further comprises a preparation of cross-linked polypeptide molecules obtainable by the method according to the present invention and/or cross-linked polypeptide molecules according to the present invention, as well as to a diagnostic test element comprising said test chemistry matrix. The present invention further relates to a preparation of cross-linked polypeptide molecules obtainable by the method according the present invention and/or cross-linked polypeptide molecules according to the present invention for use in the diagnosis of disease and for use in diagnosis of diabetes, as well as to a method for producing a test chemistry matrix.

## Description

### Field of the invention

The present invention relates to a method for cross-linking polypeptide molecules, comprising the step of combining a cross-linking agent and polypeptide molecules in solution under conditions suitable for a cross-linking reaction to occur and to a preparation comprising cross-linked polypeptide molecules obtainable by said method. The present invention further relates to cross-linked polypeptide molecules, wherein said polypeptide molecules are cross-linked by essentially unbranched cross-linking groups comprising at least 40 contiguous atoms. Moreover, the present invention relates to a test chemistry matrix comprising a redox cofactor, an agent capable of eliciting a change in at least one measurable property of an indicator reagent in the presence of redox equivalents, and an indicator reagent, wherein said chemistry matrix further comprises a preparation of cross-linked polypeptide molecules obtainable by the method according to the present invention and/or cross-linked polypeptide molecules according to the present invention, as well as to a diagnostic test element comprising said test chemistry matrix. The present invention further relates to a preparation of cross-linked polypeptide molecules obtainable by the method according the present invention and/or cross-linked polypeptide molecules according to the present invention for use in the diagnosis of disease and for use in diagnosis of diabetes, as well as to a method for producing a test chemistry matrix.

### Related art

Enzyme immobilization has been investigated in the art under the aspects of enabling recycling of enzymes used in chemical synthesis, convenience of handling (e.g. separation from the product), and of enhanced stability of immobilized enzyme preparations (reviewed in Sheldon (2007), Adv Synth Catal 349:1289). Accordingly, several methods for immobilizing enzymes have been described in the prior art, including coupling of enzymes to solid particles, crosslinking enzymes, enzyme encapsidation, or immobilizing enzymes in solid matrices (Meth Biotechnol Nr. 22: Immobilization of Enzymes and Cells, 2nd ed., J.M. Guisan (ed), Humana press). For cross-linking, enzymes are either crystallized and then cross-linked, producing cross-linked enzyme crystals (CLECs); or enzymes are precipitated and the precipitates are treated to produce cross-linked enzyme aggregates (CLEAs®) (Sheldon (2007), op. cit.). As a crosslinking agent, glutaraldehyde is generally the agent of choice. However, glutaraldehyde has the disadvantage that many enzymes are inactivated during cross-linking, which was attributed to the small size of the glutaraldehyde molecules, which are thought to penetrate into the protein molecules and to inactivate amino acids crucial for the activity of the enzyme (Sheldon (2007) op. cit.). Accordingly, dextran polyaldehyde was proposed as alternative cross-linking agent (Mateo et al. (2004), Biotech Bioeng 86(3):273), with improvements as compared to glutaraldehyde shown e.g. for nitrilases and Penicillin G acylase.

Diagnostic test elements are usually manufactured for use in near-patient applications. Therefore, the elements must be robust with respect to handling and storage. This applies, in particular, for the test chemistry of the test elements. (see Hones 2008, Diabetes Technology & Therapeutics 10: S10). However, many diagnostic test elements are based on a rather complex enzyme test chemistry present on the test element. In particular, the test element comprises a carrier and a detection layer wherein the detection layer usually contains enzymes. It is decisive for the proper function of the test elements that these enzymes remain biologically active during storage and upon treatment. Since calibration for an individual measurement is usually not possible, the test elements are normally calibrated batch-wise. The calibration information for a batch of test elements is stored and used for each element of the batch regardless of individual differences in treatment and storage.

However, pretreatments of the test elements and storage conditions can severely affect the activity of the enzymes. For example, heat treatment, either during the manufacture or during storage of the test elements, can denature the enzymes such that the overall enzymatic activity present on a test element is significantly reduced which, in turn, will result in wrong test results when such a test element is used. Similarly, with increasing water content in the detection layer, enzymes and other components of the detection layer will tend to diffuse, which is a particular problem in test elements comprising more than one layer of test chemistry. This problem is particularly pronounced in applications where test strips are exposed to environmental conditions for an extended time, e.g. in "open vial" applications or where test strips are provided in cartridges. Accordingly, enzymes comprised in such test strips have been absorbed to charged particles, e.g. to Transpafil. However, this method of immobilization has the disadvantage of severely diluting the specific activity of the enzyme immobilized, as calculated on a per mass basis, which, in turn, necessitates an increase of layer thickness in order to provide the required enzyme activity per area. This increase in layer thickness, in turn, usually leads to increased reaction times due to slowed diffusion of the analyte into the layer, meaning that increased test times are required.

Accordingly, there is a need in the art for means and methods of improving immobilization of polypeptides. In particular, methods of immobilizing enzymes avoiding strong dilution of the specific activity per mass and/or for immobilizing enzymes in the form of finely dispersible aggregates are required.

### Summary of the invention

The problems as described above are solved by means and methods with the features of the independent claims. Preferred embodiments, which might be realized in an isolated fashion or in any arbitrary combination, are listed in the dependent claims.

Accordingly, the present invention relates to a method for cross-linking polypeptide molecules, comprising the step of combining a cross-linking agent and polypeptide molecules in solution under conditions suitable for a cross-linking reaction to occur.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

The method for cross-linking polypeptide molecules of the present invention is an in vitro method. Moreover, it may comprise steps in addition to those explicitly mentioned above. For example, further steps may relate, e.g., to purifying the polypeptide prior to cross-linking and/or removal of surplus reagents and/or unwanted side products after the cross-linking step. Moreover, one or more of said steps may be performed by automated equipment.

The term "polypeptide" is known to the skilled person and relates to a chemical molecule comprising a plurality of peptide bonds. Preferably, the peptide bonds are formed between amino acids. Amino acids, preferably, are alpha-amino acids, more preferably are L-alpha-amino acids. Preferably, the polypeptide comprises at least 5 amino acids interconnected via peptide bonds; more preferably, the polypeptide comprises at least 25 amino acids interconnected via peptide bonds; most preferably, the polypeptide comprises at least 100 amino acids interconnected via peptide bonds. Preferably, the polypeptide is a polypeptide synthesized by in vitro peptide synthesis, i.e. chemical synthesis according to one of the methods known to the skilled person. More preferably, the polypeptide is a polypeptide synthesized by in vitro protein biosynthesis. Most preferably, the polypeptide is a polypeptide synthesized by in vivo protein biosynthesis, i.e. in a living cell.

Preferably, the polypeptide is a polypeptide without catalytic activity of its own, e.g. a cellular receptor, an immunoglobulin, or a part thereof. More preferably, the polypeptide is a polypeptide having catalytic activity, i.e. the polypeptide is an enzyme. It is understood by the skilled person that the purity of a polypeptide preparation is generally less than 100%. Accordingly, it is preferred that at least a fraction of the polypeptide molecules are molecules having the respective enzymatic activity; more preferably, at least 80% of the polypeptide molecules are molecules having the respective enzymatic activity; and, most preferably, at least 90% of the polypeptide molecules are molecules having the respective enzymatic activity.

Preferably, the enzyme is a dehydrogenase or comprises a dehydrogenase or oxidoreductase. The term "dehydrogenase", as used herein, refers to an enzyme which is capable of catalyzing the oxidation or reduction of a substrate by transferring hydrides (H⁻) in a one-step-mechanism or H⁺ / e⁻ in a two-step mechanism as redox equivalents to or from its redox cofactor as referred to herein elsewhere. Preferably, a dehydrogenase is a polypeptide which is capable of catalyzing the oxidation of a substrate by transferring hydrides (H⁻) as redox equivalents to an acceptor molecule, preferably, to a redox cofactor as referred to herein elsewhere. Dehydrogenases envisaged by the present invention are, preferably, those which depend on a redox cofactor (or sometimes referred to as co-enzyme) such as pyrrolo quinoline quinone (PQQ), nicotinamide-adenine-dinucleotide (NAD) or a derivative thereof, or a flavine cofactor, such as flavin-adenine-dinucleotide (FAD) or flavine mononucleotide (FMN). Preferred dehydrogenases are, in particular, lactate dehydrogenase (EC number 1.1.1.27 or 1.1.1.28), glucose dehydrogenases (see below), alcohol dehydrogenase (EC number EC number 1.1.1.1 or 1.1.1.2), L-amino acid dehydrogenase (EC number 1.4.1.5), glycerin dehydrogenase (EC number 1.1.1.6), malate dehydrogenase (EC number 1.1.1.37), 3-hydroxybutyrate dehydrogenase (EC number 1.1.1.30), sorbitol dehydrogenase (EC number 1.1.1.14), or cholesterol dehydrogenase.

More preferably, the dehydrogenase is a glucose dehydrogenase. Most preferably, said glucose dehydrogenase is selected from the group consisting of: glucose dehydrogenase (EC number 1.1.1.47), quinoprotein glucose dehydrogenase (EC number 1.1.5.2), in particular, pyrrolo quinoline quinone (PQQ)-dependent glucose dehydrogenase (EC number 1.1.5.2), glucose-6-phospate dehydrogenase (EC number 1.1.1.49), nicotinamide adenine dinucleotide (NAD)-dependent glucose dehydrogenase (EC number 1.1.1.119) and flavin adenine dinucleotide (FAD)-dependent glucose dehydrogenase (EC number 1.1.99.10) or enzymatically active mutants thereof.

Enzymatically active mutants of the aforementioned enzymes can be obtained by substituting, adding or deleting one or more amino acids from the amino acid sequences reported for the aforementioned wild type enzymes in the prior art as recited before. Preferred mutants are the mutants of the PQQ-dependent glucose dehydrogenase having an improved substrate specificity compared to their wild type counterparts as disclosed in US 7,132,270 or US 7,547,535. Further mutants are those disclosed in Baik et al (Baik 2005, Appl Environ Microbiol 71: 3285), Vasquez-Figuera et al. (Vasquez-Figuera 2007, Chem BioChem 8: 2295), and WO 2005/045016. Preferred in accordance with the present invention is a glucose dehydrogenase (E.C. 1.1.1.47) mutant disclosed in WO2009/103540A1 (p.21) or EP1660648 having a mutation at least at amino acid positions 96, 170 and/or 252, herewith incorporated by reference. Preferred mutations envisaged at theses amino acid positions are substitutions of Glu96Gly, Glul70Arg or Lys and/or Lys252Leu, the combination Glu170Lys / Lys252Leu being more preferred. Most preferably, said mutations are mutations Glu170Arg and Gln252Leu in glucose dehydrogenase from Bacillus subtilis ("GlucDH E170K/Q252L", also related to as "GlucDH2" in the Examples).

The term "cross-linking", as used herein, relates to covalently interconnecting two polypeptide molecules via an intervening cross-linking group. In principle, it is possible with the method for cross-linking polypeptide molecules according to the present invention to cross-link non-identical types of polypeptide molecules (mixed cross-link), e.g. to cross-link enzymes molecules catalyzing different steps of a chemical reaction sequence. As a non-limiting example, a glucose dehydrogenase and a diaphorase may be combined in a cross-linking reaction as described herein. Is is understood by the skilled person that mixed crosslinks are not in principle limited to two non-identical types of polypeptide molecules, but may contain more than two types of polypeptide molecules. Preferably, however, polypeptide molecules of the same molecular species are cross-linked.

The term "cross-linking agent" relates to a chemical molecule comprising reactive chemical groups forming a covalent bond with the polypeptide molecule to be cross-linked under the appropriate conditions. Preferably, the cross-linking agent comprises a contiguous chain of at least 40 atoms, more preferably of at least 50 atoms, most preferably of at least 65 atoms. Preferably, the cross-linking agent comprises at least two reactive chemical groups; more preferably, the cross-linking agent comprises exactly two reactive chemical groups. It is understood by the skilled person that at least two of the reactive chemical groups are arranged spatially in such a way to enable said two reactive chemical groups to contact two different polypeptide molecules to be cross-linked. Preferably, the cross-linking agent and/or the cross-linking group is an essentially unbranched molecule, i.e. the cross-linking agent and/or the cross-linking group comprises less than 10% of atoms constituting branch points. More preferably, the cross-linking agent is an unbranched molecule. Even more preferably, the cross-linking agent is polyethylene glycol (PEG) comprising at least one reactive chemical group as defined herein below at each end (bis-activated PEG). Most preferably, the cross-linking agent is bis-N-Hydroxy-succinimide activated polyethylene glycol (O,O'-Bis[2-(N-Succinimidyl-succinylamino)ethyl] polyethylene glycol). It is clear from the above that the cross-linking group is the backbone of the cross-linking agent remaining after at least two of the reactive chemical groups have undergone a cross-linking reaction.

Preferably, the cross-linking agent has a molecular mass of 0.6 kDa to 20 kDa, more preferably of 0.9 kDa to 10 kDa, even more preferably of 1 kDa to 5 kDa, most preferably of 3 kDa.

The term "reactive chemical group", as used herein, relates to a chemical group reacting with a group of atoms, preferably an amino acid side chain, present in a polypeptide and thereby forming a covalent bond under appropriate conditions. Preferably, the reactive chemical group is a chemical group reacting with a group of atoms present in a polypeptide under conditions not causing denaturation of said polypeptide. Preferably, said conditions not causing denaturation are physiological conditions, more preferably 25°C, 100 kPa in an appropriate solvent. Suitable reactive chemical groups are known in the art and include, preferably, cyanate esters, epoxides, isocyanates, imidates, thioimidates and , more preferably, N-hydroxy-succinimide groups.

The term "in solution", as used herein, relates to a state of a chemical compound being present in a liquid in a homogenous mixture, the chemical compound (the solute) being dissolved in the solvent. Accordingly, preferred solvents are solvents not causing the polypeptide of the present invention to denature and/or to precipitate. The skilled person knows how to select an appropriate solvent for a given polypeptide; e.g. short polypeptides can be dissolved in polar organic solvents, e.g. DMSO. Longer polypeptides, preferably comprising more than 50 amino acids, are typically best dissolved in aqueous solutions. Thus, preferably, the solution is an aqueous solution. Preferably, the solution is a buffered solution having a pH of 7 to 9, preferably 7.5 to 8.8, more preferably 8.3 ± 1, most preferably 8.3. Preferred buffers are buffers not reacting with the reactive chemical group of the cross-linking agent. More preferably, the buffer is Na- and/or K-phosphate buffer, most preferably 0.1 M K-Phosphate-buffer. It is understood by the skilled person that a specific polypeptide may require additional components in the solvent in order stay in solution, e.g. mono- and/or divalent cations, cofactors, a substrate or substrates, and/or detergents.

The skilled person understands that the selection of "conditions suitable for a cross-linking reaction to occur" depends on the cross-linking agent and in particular on the chemically reactive groups comprised therein, as well as on the specific type of polypeptide, and knows to select conditions accordingly. Preferred conditions are described elsewhere herein. Preferably, the polypeptide molecules are present in the solution at an initial concentration of 0.1 mM to 5 mM, preferably 0.3 mM to 4 mM, more preferably 2.5 ± 1 mM. Also preferably, the molar ratio of crosslinking agent / polypeptide molecules is 0.2 to 5, preferably 0.5 to 4, more preferably 0.8 to 3, most preferably 1 to 2. Preferably, combining the cross-linking agent and polypeptide molecules in solution comprises dropwise addition of the cross-linking agent to a solution comprising the polypeptide molecules. More preferably, said combining further comprises agitation of said solution comprising the polypeptide molecules. Preferably, the cross-linking agent is dissolved in an organic solvent, more preferably a compatible organic solvent, before being added to the polypeptide solution. It is understood by the skilled person that a compatible organic solvent is preferably selected such as to not react with the cross-linking agent, to not inactivate or denature the polypeptide at the final concentration present in the reaction mixture, and to dissolve the cross-linking agent. Preferably, the organic solvent is water-free. More preferably, the organic solvent is dimethylsulfoxide (DMSO). Most preferably, the organic solvent is water-free DMSO.

Advantageously, and unexpectedly, it was found in the work underlying the present invention that polypeptide molecules can be efficiently cross-linked in solution, i.e. without the need for precipitating the polypeptide. This method is advantageous in particular for enzymes which are inactivated by precipitation and for polypeptides which are difficult to re-dissolve after precipitation. Moreover, it was found that cross-linked polypeptides obtained according to the method of the present invention are especially well suited for producing test chemistry matrices, since the cross-linked polypeptides are on the one hand large enough to drastically decrease diffusion through the matrix layer or layers, and on the other hand are small enough for fine dispersion of the polypeptide over the chemistry matrix. Further, it was found that by cross-linking an enzyme according to the present invention, the dilution of specific activity which is a consequence of cross-linking with high-molecular weight polymers, can be drastically reduced.

The definitions made above apply mutatis mutandis to the following. Additional definitions and explanations made further below also apply for all embodiments described in this specification mutatis mutandis.

The present invention also relates to a preparation comprising cross-linked polypeptide molecules, obtainable by the method for cross-linking polypeptide molecules of the present invention.

Preferably, the cross-linked polypeptide molecules comprised in the preparation obtainable by the method for cross-linking polypeptide molecules of the present invention are comprised in covalent aggregates comprising on average 5 - 50 polypeptide molecules, more preferably 10 to 40 polypeptide molecules, and most preferably 15 to 25 polypeptide molecules. Accordingly, e.g. in the case of a polypeptide with a molecular mass of 1 x 10² kDa, the cross-linked polypeptide molecules, preferably, are comprised in covalent aggregates with an apparent molecular mass of 5 x 10² kDa to 5 x 10³ kDa, more preferably 1 x 10³ kDa to 4 x 10³ kDa, or, most preferably, 1.5 x 10³ kDa to 2.5 x 10³ kDa.

Preferably, the specific activity per mass of the preparation comprising cross-linked polypeptide molecules is at least 50% of the specific activity of the polypeptide before cross-linking. More preferably, the specific activity per mass of the preparation comprising cross-linked polypeptide molecules is at least 60% of the specific activity of the polypeptide before cross-linking. Most preferably, the specific activity per mass of the preparation comprising cross-linked polypeptide molecules is at least 70% of the specific activity of the polypeptide before cross-linking.

Moreover, the present invention relates to cross-linked polypeptide molecules, wherein said polypeptide molecules are cross-linked by essentially unbranched cross-linking groups comprising at least 40 contiguous atoms.

Preferably, the cross-linked polypeptide molecules are cross-linked by cross-linking groups as specified herein above. Also preferably, the cross-linked polypeptide molecules are comprised in aggregates as also specified herein above.

Further, the present invention relates to a test chemistry matrix comprising a redox cofactor, an agent capable of eliciting a change in at least one measurable property of an indicator reagent in the presence of redox equivalents, and an indicator reagent, wherein said chemistry matrix further comprises a cross-linked enzyme obtainable by the method for cross-linking polypeptide molecules of the present invention and/or cross-linked polypeptide molecules according to the present invention.

The term "matrix", as used herein, relates to a mixture comprising the compounds as specified herein. It is understood by the skilled person that the composition may comprise additional components, e.g., preferably, buffer components (e.g., of phosphate buffered saline, Tris buffer, citrate buffer, glycerine phosphate buffer, or Good's buffer) or other salts, detergents, or the like, including components as specified herein below. It will also be understood by the skilled person that the matrix of the present invention may be an inhomogeneous mixture, e.g. a dispersion or mixture.

Preferably, the matrix of the present invention is a dry composition. The term "dry composition", as used herein, means that the composition is essentially free of a solvent or a mixture of solvents. Essentially free as used herein means that at least 85%, at least 90%, at least 92%, at least 95%, at least 98%, or at least 99% of the solvent or solvent mixture which was originally present in a solution or dispersion of the composition has been removed from the composition. Accordingly, it is, preferably, envisaged that the solvent or solvent mixture is present in the dry composition of the invention in an amount of up to 5%, preferably up to 2%, more preferably up to 1%, or, most preferably, less than 1%. More preferably, a dry composition is a composition comprising water in an amount of up to 5%, preferably up to 2%, more preferably up to 1%, or, most preferably, less than 1%. Methods for determining residual water are known to the skilled person; preferably, residual water in a composition according to the present invention is determined using a phosphorus pentoxide sensor according to the method as described in WO 1993/012418. The aforementioned percentage values and the other percentage values referred herein used in order to define amounts refer to percent by weight (w/w), if not otherwise noted. Such a composition of the invention is, preferably, a solid composition under normal conditions, i.e., under room temperature and normal pressure.

The term "test chemistry matrix", as used herein, refers to a matrix of compounds comprising at least a redox cofactor, a reagent capable of eliciting a change in at least one measurable property of an indicator reagent in the presence of redox equivalents; an indicator reagent, and at least one cross-linked enzyme obtainable by the method cross-linking polypeptide molecules of the present invention and/or a preparation comprising cross-linked polypeptide molecules according to the present invention. Various possibilities of designing a test chemistry matrix are known in the art. In this regard, reference may be made to the above-mentioned prior art documents. Specifically, reference may be made to J. Hoenes et al.: The Technology Behind Glucose Meters: Test Strips, Diabetes Technology & Therapeutics, Volume 10, Supplement 1, 2008, S-10 to S-26. However, other types of test chemistry matrices are possible. Preferred compositions of the test chemistry matrix are described elsewhere herein and shown herein in the examples. Preferably, the test chemistry matrix comprises more than one type of cross-linked enzyme obtainable by the method cross-linking polypeptide molecules of the present invention and/or more than one preparation comprising cross-linked polypeptide molecules according to the present invention. E.g., preferably, it is envisaged by the present invention that the test chemistry matrix comprises a glucose dehydrogenase crosslinked according to the present invention and a diaphorase crosslinked according to the present invention, wherein the two enzymes were crosslinked in separate cross-linking reactions.

Preferably, the test chemistry matrix of the present invention is adapted to change at least one measurable property in the presence of an analyte. As used herein, the term "measurable property" relates to any property of the test chemistry which changes in the presence of the analyte and which can be transferred into a physical signal of any kind. Preferably, the change of the measurable property and/or the signal generatable therefrom are proportional to the concentration of the analyte in the sample. Preferably, the measurable property is a redox state of at least on of the components of the test chemistry matrix, preferably of a redox cofactor as described herein above and/or a redox mediator. Accordingly, preferably, the detection reaction is a redox reaction. More preferably, the detection reaction produces redox equivalents and/or electrons as intermediates and/or products. Most preferably, the measurable property is the concentration of a reduced or an oxidized redox mediator as described herein, i.e., preferably, the measurable property is the redox state of said mediator comprised in the test chemistry. Methods of converting the measurable property as defined above into a physical signal which can be read as a measurement value are well known in the art and are described e.g., in EP 0 821 234, EP 0 974 303, and US 2005/0023152.

Preferably, the measurable property of a test chemistry matrix determined is a property of a compound of the test chemistry matrix different from a mediator and/or indicator reagent; accordingly, preferably, in such case the mediator and/or indicator reagent can be omitted from the test chemisty matrix. E.g., as a non-limiting example, enzymatic reduction of NAD or a derivative thereof may be detected by UV-irradiation. Accordingly, the redox cofactor, the mediator, and the indicator agent may, preferably, be the same compound.

Preferably, the measurable property is an electrochemical property. Accordingly, the test chemistry matrix, preferably, is an electrochemical test chemistry matrix contacting at least two electrodes. Suitable electrodes, electrode setups, suitable further compounds of electrochemical test chemistry matrices and modes of operation of the same are known to the skilled person and are described, e.g. in WO 2007/071562 A1, US 2009/0198117 A1, WO 2007/071562 A1, WO 2014/001382 A1, and references cited therein. Preferred electrode configurations are disclosed, e.g., in WO2004/113910 and WO2006/027222. It is envisaged by the present invention that the electrochemical test chemistry matrix includes one or more chemical reagents for reacting with the analyte to produce an electrochemical signal that represents the presence of the analyte in the sample fluid. Preferably, electrochemical properties include amperometric or coulometric responses indicative of the concentration of the analyte. See, for example, U.S. Pat. Nos. 5,108,564, 4,919,770 and 6,054,039.

More preferably, the measurable property is an optical property. Accordingly, preferably, the test chemistry matrix performs at least one optically detectable detection reaction in the presence of the analyte. Still more preferably, the optically detectable signal produced by the detection reaction is proportional to the amount and/or to the concentration of the analyte in the sample. Preferably, the measurable property is a change in color and/or in color intensity of the test chemistry, i.e., preferably, a change in the absorption and/or emission spectrum of the test chemistry. Thus, in the change of the measurable property the optical property preferably is selected from the group consisting of: a reflection property, preferably a reflectance and/or a remission; transmission property, preferably an absorption; a color; a luminescence, preferably a fluorescence.

The term "detecting" relates to the quantification of the amount of analyte present in a sample of a body fluid, i.e. measuring the amount or concentration of said analyte, preferably semi-quantitatively or quantitatively. The detection of the amount of the analyte can be accomplished in a variety of ways known to the skilled person or detailed herein below. In accordance with the present invention, detecting the amount of the analyte can be achieved by all known means for detecting the amount of said analyte in a sample, provided that they are adapted to specifically detect the analyte of the present invention and are compatible with the requirements of the present invention. The term "amount" as used herein encompasses the absolute amount of the analyte referred to herein, the relative amount or concentration of the analyte referred to herein as well as any value or parameter which correlates thereto. Such values or parameters comprise intensity signal values from all specific physical or chemical properties obtained from the analyte referred to herein by measurements. It is to be understood that values correlating to the aforementioned amounts or parameters can also be obtained by all standard mathematical operations.

The term "redox cofactor", as used herein, refers to a molecule which can serve as an acceptor for enzymatically transferred redox equivalents and, in particular, hydride (H⁻). Preferably, the redox cofactor is PQQ, NAD or FAD. It will be understood that the redox cofactor to be included in the test chemistry matrix of the present invention depends on the properties of the dehydrogenase to be envisaged. For example, PQQ is combined in a composition according to the present invention with a PQQ dependent glucose dehydrogenase, NAD is combined in a composition according to the present invention with a NAD dependent glucose dehydrogenase, and FAD is combined in a composition according to the present invention with a FAD dependent glucose dehydrogenase. A redox cofactor according to the present invention may also preferably be a derivative of PQQ, NAD or FAD. Preferred derivatives of NAD are those disclosed in WO 2007/012494 which is herewith incorporated by reference with respect to the disclosed NAD/NADH and/or NADP/NADPH derivatives. More preferably, the redox cofactor in accordance with the present invention is carbaNAD as disclosed in WO 2007/012494.

Accordingly, the term "agent capable of eliciting a change in at least one measurable property of an indicator reagent in the presence of redox equivalents" refers to a molecule which, in the presence of redox equivalents, is capable of inducing a change in at least one measurable property in an indicator reagent. It is to be understood in accordance with the present invention that the agent may also elicit a change in more than one measurable property of the indicator reagent which may then subsequently be detected. Furthermore, the agent may, preferably, also elicit a change in measurable properties of more than one indicator reagent which may then subsequently be detected.

An agent as referred to above is, preferably, capable of transferring directly or indirectly, i.e., via a further mediator, redox equivalents from the redox cofactor to the indicator reagent. As a consequence of the said transfer of the redox equivalents, the indicator reagent will be modified such that a change in at least one measurable property occurs. In case the measurable property is an optical property, preferably, for example, a color-less or non-fluorescing indicator reagent in an oxidized state may be converted into a colored or fluorescent indicator reagent by the transfer of redox equivalents mediated by the agent in a reduced state. The transfer of the redox equivalents may be direct in that the redox equivalents are transferred by the agent to the indicator reagent or may be indirect. In the latter case, the redox equivalents are transferred from the agent to an intermediate mediator which subsequently transfers the redox equivalents to the indicator reagent. It will be understood that, preferably, more than one mediator can be used. For example, the agent may transfer the redox equivalents to a first mediator which subsequently transfers the redox equivalents to a second mediator and said second mediator then transfers the redox equivalents to the indicator reagent. It will be understood that in such a mediator cascade more than two mediators could be used. An advantage of using one or more mediators for the transfer of the redox equivalents to the indicator reagent is that the timing of the detection of the measurable property can be improved.

Mediators which can be applied in the context of the present invention are well known in the art and include, e.g., potassium ferricyanide, quinone derivatives, Nile blue (CAS no.: 3625-57-8), Meldola's blue (CAS no.: 7057-57-0), osmium complexes as disclosed in EP 1 457 572 B1, transition metal complexes such as ruthenium hexamine chloride, or nitroso-aniline-derivatives

An agent according to the invention which is, preferably, envisaged is a phenazine. More preferably, said phenazine is phenazinethosulfate, phenazinmethosulfate, 1-(3-carboxypropoxy)-5-ethylphenaziniumtrifluoromethansulfonate or 1-methoxyphenazine-methosulfate. Such phenazines can be applied for eliciting a change in at least one optical property of an indicator reagent. Details for the detection and on how such phenazines are to be applied can be found in EP 0 654 079 A1. Also, an agent envisaged in this context is, preferably, a chinone. More preferably, the said chinone is phenanthrenchinone, phenanthrolinchinone or benzo[h]-chinolinchinone. Another agent envisaged in this context is a nitrosoaniline. More preferably, said nitrosoaniline is [(4-nitrosophenyl)imino]dimethanol-hydrochloride. Most preferably, said nitrosoaniline is 4-Bis(hydroxyethyl)-1-nitroso-aniline or 4-Bis(hydroxyethyl)-3-methoxy-1-nitrosoaniline. Also preferably envisaged by the present invention is an agent capable of eliciting a change in at least one measurable property of an indicator reagent in the presence of redox equivalents, said agent being an enzyme capable of catalyzing the transfer of redox equivalents from the redox cofactor to the indicator reagent.

The term "indicator reagent", as used herein, preferably, relates to a compound changing at least one measurable, preferably optical, property dependent on, preferably proportional to, the activity of the enzyme of the present invention. Preferably, the indicator reagent is an optical indicator substance, which performs at least one optically detectable property change when at least one of the enzymes or when the enzyme comprised in the test chemistry matrix reacts with the analyte. Thus, the at least one indicator reagent preferably comprises one or more dyes performing a change in an optical property indicative of the enzymatic reaction of the at least one enzyme and the analyte. The optical property of the indicator reagent, according to the present invention, changes dependent on the activity of the enzyme of the present invention. Thus, preferably, the change of the optical property only occurs if the enzyme catalyzes the detection reaction. More preferably, the change of optical property is proportional to the number of catalytic cycles undergone by the enzyme present in the test chemistry matrix. Thus, most preferably, the change of optical property is proportional to the number of analyte molecules converted by the enzyme.

Preferably, the optical property changing in the indicator reagent is measurable in the test chemistry matrix comprising said indicator reagent. Thus, the at least one optical property may be any property of the test chemistry matrix which changes in the presence of the analyte and which can be transferred into an optical signal of any kind. Preferably, the change of the optical property and/or the signal generatable therefrom are proportional to the concentration of the analyte in the sample. Additionally or alternatively, a predetermined or determinable relationship between the optical property, the change of the optical property and/or the signal generatable therefrom and the concentration of the analyte in the sample may exist which may be used for deriving the concentration from the signal.

It is understood by the skilled person that the measurable property of a test chemistry matrix determined may also be a property of a compound of the test chemistry matrix different from a mediator and/or indicator reagent and that, in such case, the mediator and/or indicator reagent can be omitted from the test chemisty matrix; e.g., preferably, enzymatic reduction of NAD or a derivative thereof may be detected by UV-irradiation. Accordingly, the redox cofactor, the mediator, and the indicator agent may, preferably, be the same compound.

The term "analyte", as used herein, relates to a chemical compound present in a body fluid. Preferably, the analyte is a small molecule, i.e., preferably, the analyte is not a biological macromolecule. More preferably, the analyte is an organic molecule, most preferably an organic molecule capable of undergoing a redox reaction in the presence of the test chemistry according to the present invention. Preferably, the analyte is a molecule of the subject's metabolism. Also preferably, the analyte is a low molecular weight chemical compound, more preferably a chemical compound with a molecular mass of less than 1000 u (1000 Da; 1.66×10⁻²⁴ kg). More preferably, the analyte is selected from the list consisting of malate, ethanol, ascorbic acid, cholesterol, glycerol, urea, 3-hydroxybutyrate, lactate, pyruvate, triglycerides, ketones, liver parameters, creatinine, HDL, and the like; more preferably, the analyte is blood glucose. Preferably, the analyte is blood glucose and the actual concentration to be determined is at least 10 mg/dL, at least 50 mg/dL, at least 60 mg/dL, at least 70 mg/dL, at least 80 mg/dL, at least 90 mg/dL, at least 100 mg/dL, at least 110 mg/dL, at least 120 mg/dL, at least 130 mg/dL, at least 140 mg/dL, or at least 150 mg/dL. Preferably, the analyte is glucose and the the concentration to be determined is in the range 0 mg/dL to 800 mg/dL, or, more preferably, 10 mg/dL to 600 mg/dL, or, most preferably, 50 mg/dL to 300 mg/dL.

As used herein, the term "body fluid" relates to all bodily fluids of a subject known to comprise or suspected to comprise the analyte of the present invention, including blood, plasma, serum, lacrimal fluid, urine, lymph, cerebrospinal fluid, bile, stool, sweat, and saliva. Preferably, the body fluid is blood. The term "sample" is understood by the skilled person and relates to any subportion of a bodily fluid, preferably removed from the subject prior to applying said sample to a test element. Samples can be obtained by well known techniques including, e.g., venous or arterial puncture, epidermal puncture, and the like.

Further, the present invention relates to a method for manufacturing a test chemistry matrix, comprising the steps of
a) obtaining a preparation of cross-linked enzyme molecules according to the method for cross-linking polypeptide molecules of the present invention and/or cross-linked polypeptide molecules according to the present invention, and
b) admixing a redox cofactor, an agent capable of eliciting a change in at least one measurable property of an indicator reagent in the presence of redox equivalents, an indicator reagent, and the preparation of cross-linked enzyme molecules obtained in step a), and, thereby,
c) producing a test chemistry matrix.

Moreover, the present invention relates to a diagnostic test element for the determination of an analyte from a body fluid sample comprising the test chemistry matrix of the present invention on a carrier.

The term "carrier", as used in the context of the present invention, refers to a solid support onto which the test chemistry matrix of the present invention is applied. Preferably, the test chemistry matrix can be immobilized on the carrier. Moreover, it is also envisaged that the test chemistry matrix can be spatially arranged on the carrier. The carrier must be arranged in a manner as to allow for the detection of the change of the at least one measurable property of the indicator reagent, i.e., it preferably does not comprise components or a spatial arrangement which would interfere with the detection of the at least one measurable property. Suitable carriers may comprise vials containing the composition of the present invention, e.g., vials arranged in a well-plate format. Other assays may apply optical waveguides or semiconductor plates. Preferred carriers, however, are those used for test strips. Said test strips usually comprise one or more layers forming a solid carrier.

Preferably, the diagnostic test element of the invention comprises a test field containing said test chemistry matrix, wherein the test field has a sample application side onto which the body fluid sample is applied and a detection side which allows for detection of a change in a measurable property when the analyte reacts with the components of the test chemistry matrix. The sample is to be applied to the sample application side and it is, preferably, envisaged that cells, such as erythrocytes present in blood samples, do not reach the detection side. Details on such a diagnostic test element and the manufacture thereof can be found in EP 0 821 234 B1. Further test elements envisaged in accordance with the present invention are those disclosed in EP 1 035 919 B1 or EP 1 035 920 B1.

The test field of the diagnostic test element of the present invention comprises, preferably, a transparent foil onto which one, more preferably more than one film layer are applied. The film layers of the diagnostic test element are produced from dispersions or emulsions of polymeric film formers. Dispersion film formers contain microscopic polymer particles which are insoluble in the carrier liquid (usually water) and are finely dispersed in the carrier liquid. If the liquid is removed by evaporation during film formation then the particles come closer and finely touch one another. The large forces which occur in this process and the gain in surface energy which accompanies the film formation results in the particles growing into a substantially closed film layer. Alternatively, it is also possible to use an emulsion of the film former in which this is dissolved in a solvent. The dissolved polymer is emulsified in a carrier liquid which is immiscible with the solvent. Polyvinyl esters, polyvinyl acetates, polyacrylic esters, polymethacrylic acid, polyacrylamides, polyamides and polystyrene are particularly suitable as polymers for such film formers. In addition to homopolymers mixed polymerizates are also suitable such as of butadiene, styrene or maleic acid ester.

By adding a swelling agent that swells well (i.e., a substance which increases its volume when it takes up water) one does not only obtain layers which can be penetrated relatively rapidly by sample liquid but which also have good cell, e.g., erythrocyte and additionally also blood pigment, separation properties despite this opening effect of the swelling agent. The swelling properties should be so good that for a test in which the change of the at least one measurable property is mainly dependent on the penetration of the sample liquid through the layer, the change of the measurable property is measurable after a maximum of one minute. Especially suitable swelling agents have proven to be methyl vinyl ether maleic acid anhydride copolymer, xanthan gum and methyl vinyl ether maleic acid copolymer.

Single layer layouts of diagnostic test elements are known from the prior art, see, e.g., EP 1 566 637 and EP 1 780 288. More preferred two-layer layouts, preferably, comprise a first and a second film layer resting on top of one another in this order. It is important that the first layer located on the transparent foil scatters light considerably less than the overlying second layer. The non-coated side of the transparent foil is referred to as the detection side and the side of the second layer which is opposite to the side with which the second layer rests on the first layer is referred to as the sample application side. The two so-called film layers are located on a transparent foil in the test field of the diagnostic test carrier according to the invention. For this those plastic foils come into consideration which are impermeable to liquid. Polycarbonate foil has proven to be particularly suitable. The two film layers can be produced from coating compounds which contain the same polymeric film formers or they can be produced from coating compounds which contain different polymeric film formers. Whereas the first layer contains a swelling agent and optionally a weakly light scattering filler, the second layer requires a swelling agent and in any case at least one pigment that scatters light strongly. In addition the second layer can also contain non-porous fillers as well as porous fillers.

In the case of a single-layer film, which may, preferably, be used in electrochemical detection, the cross-linked polypeptide of the present invention is comprised in said single layer. In the case of multi-layer (i.e., more than one layer) layouts, it is preferred that the cross-linked polypeptide of the present invention is comprised in one film layer, more preferably the first film layer.

In order to optimize the test field in the diagnostic test element according to the invention, it has proven to be particularly advantageous when all film layers, preferably both layers in the case of two-layer layouts, do contain a non-haemolyzing wetting agent. Neutral, i.e., non-charged wetting agents are particularly preferred for this. N-octanoyl-N-methyl glucamide is most particularly preferred.

In order to produce a two-layer test field of a diagnostic test element according to the invention, the respective film layers are each produced successively from a homogeneous dispersion of the said components. For this, the transparent foil is used as a base to form the coating compound for the first film layer. After the coating compound for the first film layer has been applied with a particular layer thickness, the layer is dried. Afterwards the coating compound for the second layer is applied to this layer also with a thin layer thickness and dried. The test field produced in this manner can be mounted on a supporting layer for better handling, those materials coming into consideration for such a layer which do not take up the liquid to be examined. These are so-called non-absorptive materials, plastic foils for example made of polystyrene, polyvinyl chloride, polyester, polycarbonate or polyamide being particularly preferred. Metal foils or glass are suitable as further supporting materials.

In a preferred embodiment of the test element according to the invention, the detection side of the test field which is to be observed and measured for a change in at least one optical property of the indicator reagent should be visible through the supporting layer in order to determine the analyte to be detected in the body sample. This can be achieved by a transparent supporting layer. However, it is also possible that the supporting layer has a perforation which is covered by the detection side of the test field. The detection side is then visible through the perforation. Preferably, in the diagnostic test element according to the invention there is a hole in the supporting layer below the detection side of the test field through which the detection side of the test field can be observed. The hole has a somewhat smaller diameter than the smallest linear dimension of the test field so that the test field outside the hole rests on the supporting layer and can be attached there.

In another preferred embodiment of the test element according to the present invention, the test element comprises a capillary canal. Such a layout is known from the prior art, e.g., from EP 1 035 921. In a further preferred embodiment, the test element according to the present invention is produced from a test element band, see e.g., EP 1 593 434 or is a test strip. Moreover, in a further preferred embodiment, the diagnostic test element is comprised in a cartridge, said cartridge preferably comprising a multitude of diagnostic test elements.

The present invention further relates to a preparation comprising cross-linked polypeptide molecules according to the present invention and/or cross-linked polypeptide molecules according to the present invention for use in the diagnosis of disease. Accordingly, the present invention relates to a preparation of cross-linked polypeptide molecules obtainable according to the method for cross-linking polypeptide molecules for use in the diagnosis of disease; and to cross-linked polypeptide molecules, wherein said polypeptide molecules are cross-linked by unbranched side chains comprising at least 40 contiguous atoms according to the present invention, for use in the diagnosis of disease.

The term "diagnosis", as used herein, refers to assessing the probability according to which a subject is suffering or will suffer from a disease or condition referred to in this specification. As will be understood by those skilled in the art, such an assessment is usually not intended to be correct for 100% of the subjects to be diagnosed. The term, however, requires that a statistically significant portion of subjects can be correctly diagnosed to suffer from the disease or condition. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98% or at least 99%. The p-values are, preferably, 0.1, 0.05, 0.01, 0.005, or 0.0001. Preferably, the probability envisaged by the present invention allows that the diagnosis will be correct for at least 60%, at least 70%, at least 80%, or at least 90% of the subjects of a given cohort or population.

As used herein, the term "disease" relates to any clinically relevant deviation from normal metabolism. Accordingly, "diagnosis of disease" relates to determining at least one clinically relevant parameter and/or emergency parameter, including, without limitation, malate, ethanol, ascorbic acid, cholesterol, glycerol, urea, 3-hydroxybutyrate, lactate, pyruvate, triglycerides, ketones, liver parameters, creatinine, HDL, and the like and, preferably, evaluating whether a clinically relevant deviation from normal metabolism is present. It is understood by the skilled person that diagnosis, preferably, includes determining the absence of said clinically relevant deviation, i.e., preferably, diagnosis by exclusion.

Moreover, the present invention relates to a preparation comprising cross-linked polypeptide molecules according to the present invention and/or cross-linked polypeptide molecules according to the present invention for use in the diagnosis of diabetes. Accordingly, the present invention relates to a preparation of cross-linked polypeptide molecules obtainable according to the method for cross-linking polypeptide molecules for use in the diagnosis of diabetes; and to cross-linked polypeptide molecules, wherein said polypeptide molecules are cross-linked by unbranched side chains comprising at least 40 contiguous atoms according to the present invention for use in the diagnosis of diabetes.

The present invention also relates to a use of the cross-linked polypeptide molecules obtainable by the method according the present invention and/or of cross-linked polypeptide molecules according the present invention for the manufacture of a test chemistry matrix according to the present invention and/or of a diagnostic test element according to the present invention.

Moreover, the present invention relates to a use of the cross-linked polypeptide molecules obtainable by the method according the present invention and/or of cross-linked polypeptide molecules according the present invention for the manufacture of a diagnostic composition. Further, the present invention relates to a method for the manufacture of a diagnostic test element comprising the step of generating a test chemistry matrix of the present invention on a carrier.

All references cited in this specification are herewith incorporated by reference with respect to their entire disclosure content and the disclosure content specifically mentioned in this specification.

Summarizing the findings of the present invention, the following embodiments are preferred:
Embodiment 1: A method for cross-linking polypeptide molecules, comprising the step of combining a cross-linking agent and polypeptide molecules in solution under conditions suitable for a cross-linking reaction to occur.
Embodiment 2: The method of embodiment 1, wherein the polypeptide is an enzyme.
Embodiment 3: The method of embodiment 1 or 2, wherein the cross-linking agent comprises a contiguous chain of at least 40 atoms.
Embodiment 4: The method of any one of embodiments 1 to 3, wherein the cross-linking agent is an unbranched molecule chemically activated at both ends.
Embodiment 5: The method of any one of embodiments 1 to 4, wherein the cross-linking agent is bis-activated polyethylene glycol.
Embodiment 6: The method of any one of embodiments 1 to 5, wherein the cross-linking agent is bis-N-Hydroxy-succinimide activated polyethylene glycol (O,O'-Bis[2-(N-Succinimidyl-succinylamino)ethyl] polyethylene glycol).
Embodiment 7: The method of any one of embodiments 1 to 6, wherein the cross-linking agent has a molecular mass of 0.6 kDa to 20 kDa, preferably 0.9 kDa to 10 kDa, more preferably 1 kDa to 5 kDa, most preferably 3 kDa.
Embodiment 8: The method of any one of embodiments 1 to 7, wherein the polypeptide molecules are present in said solution at an initial concentration of 0.1 mM to 5 mM, preferably 0.3 mM to 4 mM, more preferably 2.5 ± 1 mM.
Embodiment 9: The method of any one of embodiments 1 to 8, wherein the molar ratio of crosslinking agent / polypeptide molecules is 0.2 to 5, preferably 0.5 to 4, more preferably 0.8 to 3, most preferably 1 to 2.
Embodiment 10: The method of any one of embodiments 1 to 9, wherein said solution has a pH of 7 to 9, preferably 7.5 to 8.8, more preferably 8.3 ± 1, most preferably 8.3.
Embodiment 11: The method of any one of embodiments 1 to 10, wherein at least a fraction of the polypeptide molecules are dehydrogenase molecules, preferably wherein at least 80% of the polypeptide molecules are dehydrogenase molecules, more preferably wherein at least 90% of the polypeptide molecules are dehydrogenase molecules.
Embodiment 12: The method of any one of embodiments 1 to 11, wherein at least a fraction of the polypeptide molecules are glucose dehydrogenase molecules, preferably wherein at least 80% of the polypeptide molecules are glucose dehydrogenase molecules, more preferably wherein at least 90% of the polypeptide molecules are glucose dehydrogenase molecules.
Embodiment 13: The method of any one of embodiments 1 to 12, wherein at least a fraction of the polypeptide molecules are GlucDH E170K/Q252L molecules, preferably wherein at least 80% of the polypeptide molecules are GlucDH E170K/Q252L molecules, more preferably wherein at least 90% of the polypeptide molecules are GlucDH E170K/Q252L molecules.
Embodiment 14: The method of any one of embodiments 1 to 13, wherein combining a cross-linking agent and polypeptide molecules in solution comprises dropwise addition of the cross-linking agent to a solution comprising the polypeptide molecules, preferably under agitation.
Embodiment 15: The method of any one of embodiments 1 to 14, wherein combining a cross-linking agent and polypeptide molecules in solution comprises dropwise addition of the cross-linking agent dissolved in a compatible organic solvent to a solution comprising the polypeptide molecules.
Embodiment 16: The method of embodiment 15, wherein the compatible organic solvent is dimethylsulfoxide (DMSO).
Embodiment 17: A preparation comprising cross-linked polypeptide molecules, obtainable by the method of any one of embodiments 1 to 16.
Embodiment 18: Cross-linked polypeptide molecules, wherein said polypeptide molecules are cross-linked by essentially unbranched cross-linking groups comprising at least 40 contiguous atoms.
Embodiment 19: The cross-linked polypeptide molecules of embodiment 18, wherein the polypeptide is an enzyme, preferably a dehydrogenase.
Embodiment 20: The cross-linked polypeptide molecules of embodiment 18 or 19, wherein the polypeptide is a glucose dehydrogenase, preferably GlucDH E170K/Q252L.
Embodiment 21: The cross-linked polypeptide molecules of any one of embodiments 18 to 20, wherein said cross-linking groups are polyethylene glycol chains.
Embodiment 22: A test chemistry matrix comprising a redox cofactor, an agent capable of eliciting a change in at least one measurable property of an indicator reagent in the presence of redox equivalents, and an indicator reagent, wherein said chemistry matrix further comprises a cross-linked enzyme obtainable by the method according to any one of embodiments 1 to 16 and/or cross-linked polypeptide molecules according to any one of embodiments 19 to 21.
Embodiment 23: The test chemistry matrix of embodiment 22, wherein said redox cofactor, said agent capable of eliciting a change in at least one measurable property of an indicator reagent, and/or said indicator agent are the same compound.
Embodiment 24: A method for producing a test chemistry matrix, comprising the steps of
   a) obtaining a preparation of cross-linked enzyme molecules according to the method of any one of embodiments 2 to 16 and/or cross-linked enzyme molecules according to any one of embodiments 19 to 21, and
   b) admixing a redox cofactor, an agent capable of eliciting a change in at least one measurable property of an indicator reagent in the presence of redox equivalents, an indicator reagent, and the preparation of cross-linked enzyme molecules obtained in step a), and, thereby,
   c) producing a test chemistry matrix.
Embodiment 25: The methods of embodiment 24, wherein said redox cofactor, said agent capable of eliciting a change in at least one measurable property of an indicator reagent, and/or said indicator agent are the same compound.
Embodiment 26: A diagnostic test element for the determination of an analyte from a body fluid sample comprising the test chemistry matrix of embodiment 22 or 23 on a carrier.
Embodiment 27: The diagnostic test element of embodiment 26, wherein at least a part of the test chemistry matrix is comprised in a test field.
Embodiment 28: The diagnostic test element of embodiment 26 or 27, wherein the diagnostic test element is a test strip or a test band.
Embodiment 29: The diagnostic test element of any one of embodiments 26 to 28, comprised in a cartridge.
Embodiment 30: A preparation comprising cross-linked polypeptide molecules according to embodiment 17 and/or cross-linked polypeptide molecules according to any one of embodiments 18 to 21 for use in the diagnosis of disease.
Embodiment 31: A preparation comprising cross-linked polypeptide molecules according to embodiment 17 and/or cross-linked polypeptide molecules according to any one of embodiments 18 to 21 for use in the diagnosis of diabetes.
Embodiment 32: Use of a cross-linked polypeptide obtainable by the method according to any one of embodiments 1 to 16 and/or of a preparation according to any one of embodiments 18 to 21 for the manufacture of a test chemistry matrix according to embodiment 22 or 23 and/or of a diagnostic test element according to any one of embodiments 26 to 29.
Embodiment 33: Use of a cross-linked polypeptide obtainable by the method according to any one of embodiments 1 to 16 and/or the preparation according to any one of embodiments 18 to 21 for the manufacture of a diagnostic composition.
Embodiment 34: A method for the manufacture of a diagnostic test element comprising the step of generating a test chemistry matrix according embodiment 22 or 23 on a carrier.

### Short description of the Figures

Further optional features and embodiments of the invention will be disclosed in more detail in the subsequent description of preferred embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures.

### In the Figures:

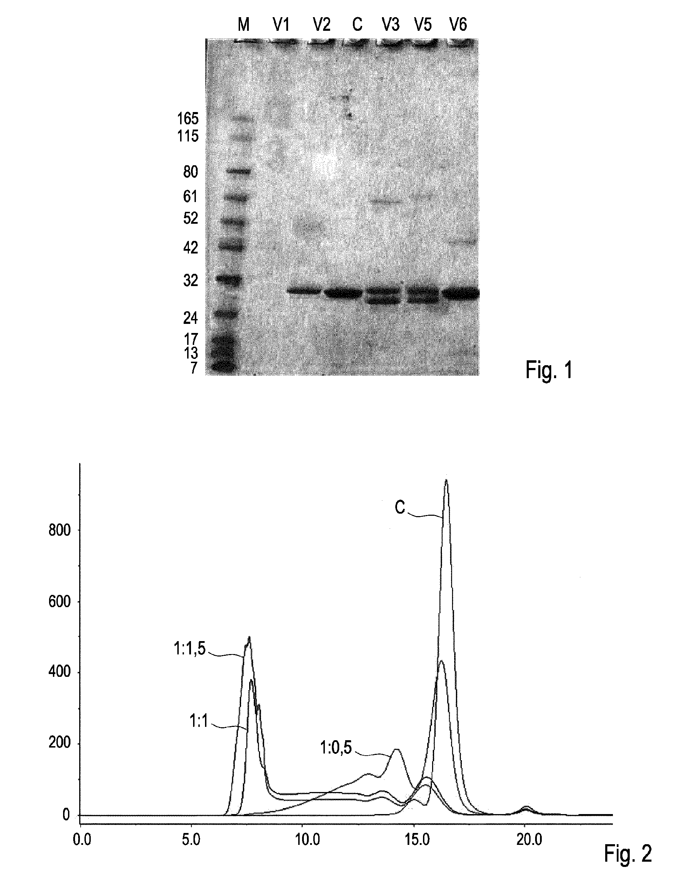

Figure 1: SDS-gel-chromatographic analysis of cross-linking products as specified in Table 1 (acrylamide concentration in the gel 4 to 12 %); amphiblue staining of GlucDH2. in lane V1 corresponding to experiment V1, no free GlucDH2 is found. M: marker; C: control; V1 - V6: lanes corresponding to experiments V1 - V6 of Table 1. The arrow indicates the position of the GlucDH2 monomer. V1 and V2 were cross-linked with NHS PEG; V3, V5 and V6 were cross-linked with expoxy-PEG. The molecular weight of marker compounds is indicated in kDa.
Figure 2: Gel permeation chromatography of reaction products of Experiment 2. Reaction products were chromatographed on a superpose 6 10/300 column with a mobile phase of 150 mM potassium phosphate buffer/150 mM NaCl, pH 7.0, at a flow rate of 0.5 ml/min and were detected in a flow cell at 280 nm. The amount of sample was 1.25 mg of protein in each case. X-axis: elutio volume (ml); Y-axis: absorbance at 280 nm (arbitrary units).

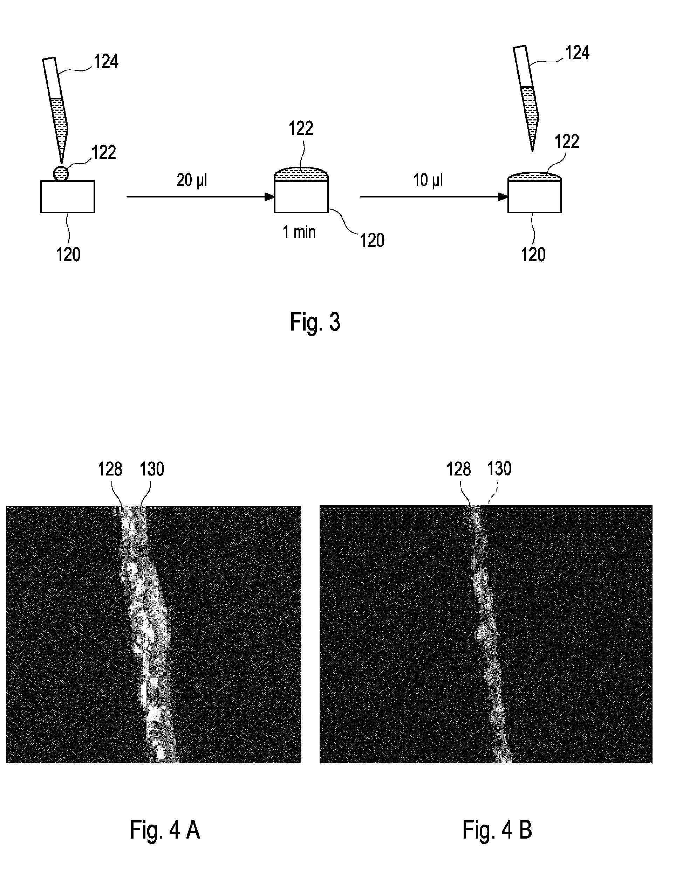

Figure 3: Schematic representation of the steps of the diffusion test: 20 µl of buffer 122 are pipetted onto a glucose test strip 120 with the help of a pipet 124. After one minute, 10 µl of the buffer 122 are withdrawn into the pipet 124 and used for further analysis.
Figure 4: Fluorescence-microscopic pictures of dry test strips comprising fluorescently labeled enzyme directly after producing the layers. A: free GlucDH2 B: cross-linked GlucDH2. In Figure 1A, both the first layer 128, into which the labelled enzyme was originally included, and the second layer 130, into which the labeled enzyme has diffused, are visible. In Figure 1 B, only the first layer 128, into which the labelled enzyme was originally incorporated, is visible.

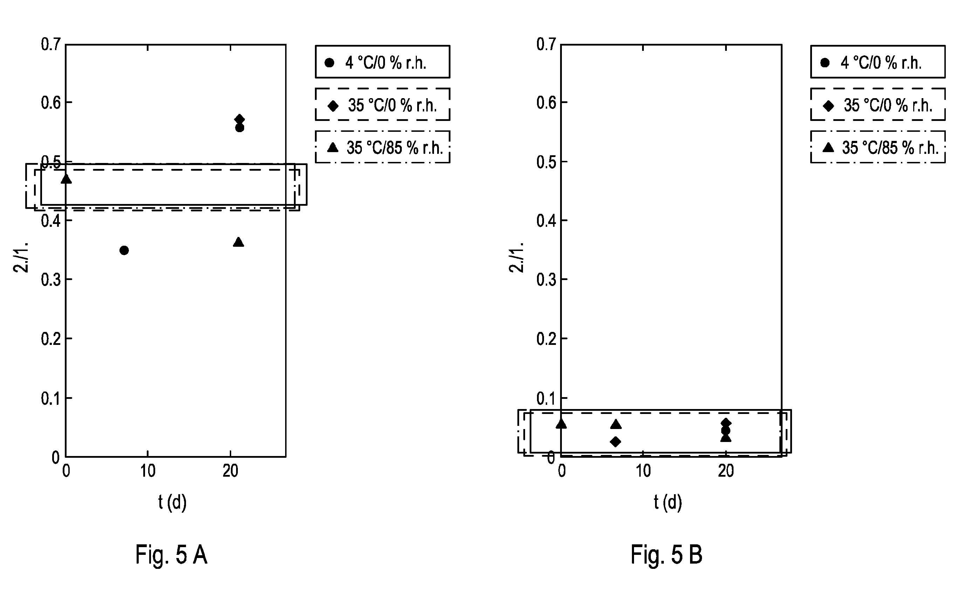

Figure 5: Quantitative evaluation of the fluorescence-microscopic evaluation of the diffusion of the fluorescent signal between the first and second layer of the test strip while stored under various conditions. X-axis: Incubation time (days); Y-axis: Ratio of amount of enzyme found in the second layer vs. amount of enzyme found in first layer. A: free GlucDH2; B: cross-linked GlucDH2.

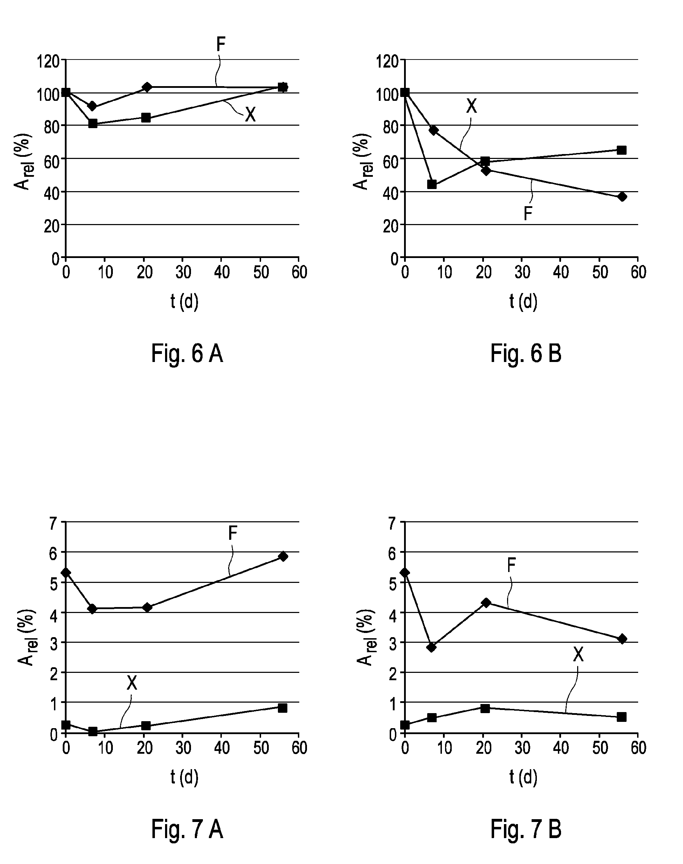

Figure 6: Stability of GlucDH2 under various storage conditions F: free enzyme; X: cross-linked enzyme; A: dry and warm conditions; B: moist and warm conditions. X-axis: Incubation time (days); Y-axis: Residual activity (Aᵣₑₗ) relative to time = 0 days (%).
Figure 7: Mobility of GlucDH2 after storage under dry and warm or moist and warm conditions. F: free GlucDH2; X: cross-linked GlucDH2. A: Storage under dry and warm conditions; B: Storage under moist and warm conditions. X-axis: Incubation time (days); Y-axis: Percent mobility (%).

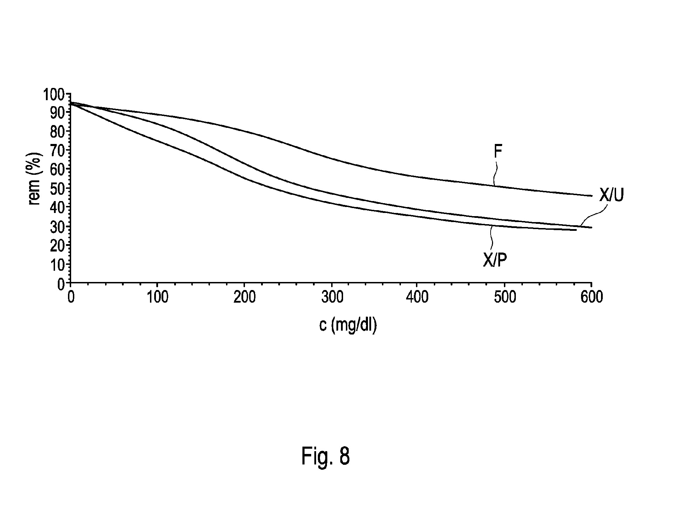

Figure 8: Performance of GlucDH2 at various glucose concentrations. In each case, relative remission in dependence of glucose concentration of a glucose test strip is indicated. X-axis: Glucose concentration in mg/dL; Y-axis: relative remission (%). F: free GlucDH2; X/U: cross-linked GlucDH2, unpurified (comprising a fraction of free enzyme), X/P: cross-linked GlucDH2, purified (devoid of free enzyme).

### Examples

### Example 1:

In a first attempt to cross-link GlucDH2, short-chain Bis-expoxy- and Bis-NHS-activated polyethylene glycols (PEGs) were used, using the following ratios: 0.3 mM GlucDH2 (in a 10 mg/ml protein solution) in reaction with a tenfold excess of Bis-NHS-activated PEGs or a 50fold excess of Bis-expoxy-activated PEG. Reactions were performed in 0.1 molar potassium phosphate buffer at pH 7.5, adding the activated PEGs dropwise at room temperature under stirring. Reactions with Bis-expoxy-activated PEG led to a complete loss of activity of GlucDH2 at low molecular masses (380 and 520 g/mol), whereas with an intermediate molecular mass (980 g/mol) 85 % of the activity could be preserved. This experiment shows which lowest molecular mass is required for an activated linker in order to prevent the essential lysine residue in GlucDH2 from reacting, which would lead to a complete inactivation of the enzyme. However, no high-molecular weight protein aggregates could be obtained by cross-linking with expoxy-activated PEG (Figure 1). In contrast, NHS-activated PEGs with molecular masses of 3,000 and 10,000 g/mol showed preservation of activity as well as cross-linking (Table 1, Figures 1 and 2). The products of cross-linking with 3,000 g/mol Bis-NHS-activated PEG were analyzed by gel permeation chromatography, using a superose 6-column. It was found that the conditions indicated above mainly led to PEGylation instead of cross-link.

**Table 1**

| ***experiment*** | ***crosslinker (structure)*** | ***Mol Wt PEG (g*/*mol)*** | ***Residual activity(%)*** | ***result*** |
|---|---|---|---|---|
| ***V1*** | 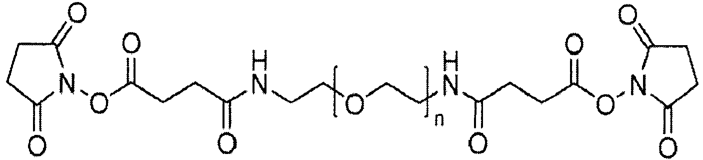 | *3000* | *61%* | *cross-linked and active enzyme*, *no monomers* |
| ***V2*** | 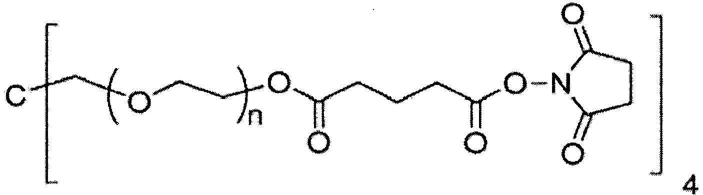 | *10000* | *90%* | *cross-linked and active enzyme*, *small amounts of monomers* |
| ***V3*** | 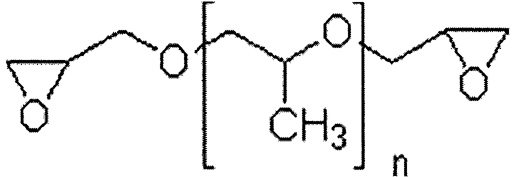 | *380* | *1*% | *inactive enzyme, no cross-link* |
| ***V5*** | 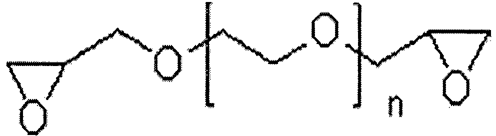 | *526* | *9*% | *inactive enzyme*, *no cross-link* |
| ***V6*** | 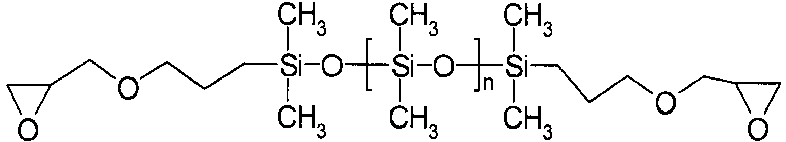 | *980* | *85%* | *active enzyme*, *no cross-link* |

**Table 2:**

| ***molar ratio*** | ***1 : 0*** | ***1 : 0,5*** | ***1 : 1*** | ***1 : 1,5*** | ***1 : 2*** |
|---|---|---|---|---|---|
| ***conc. of PEG for 2,85 mM GlucDH2*** | *0* | *1,42 mM* | *2,85 mM* | *4,2 mM* | *5,7 mM* |
| ***mol. wt via GPC (kDa)*** | *112* | *112-250* | *250-1000* | *1000-2000* | *no GPC* |
| ***%Mobility(supernatanttest)*** | *100%* | *73%* | *14,5%* | *6,8*% | *1,4*% |
| ***Stability in a test strip* (*% residual activity, 1 week, 35°C, 85 % rel. hum.)*** | *72*% | *90%* | *81%* | *119%* | *111*% |

### Example 2:

In order to optimize cross-linking, the amount of protein was drastically increased in order to increase intermolecular reaction. The pH value was also increased in order to improve the reactivity of amino groups comprised in the enzyme towards NHS-groups. Moreover, NHS-activated PEGs were dissolved in Dimethylsulfoxid (DMSO) in order to avoid premature hydrolysis of the NHS group. Accordingly, the following improved reaction conditions were used: 2.85 mM GlucDH2 (80 mg/ml protein solution) reacting with 0.5 to 2fold excess of Bis-NHS-activated PEG (3,000 g/mol). Cross-link was performed in 0.2 molar sodium-bicarbonate buffer at pH 8.3 adding NHS-activated PEG dissolved in DMSO by slow dropping at room termperature while stirring the reaction mixture. The resulting, viscous solution was dialyzed against 0.05 molar potassium/sodium phosphate buffer pH 7.0. Activity was determined by an activity assay and the size of the products was determined by gel permeation chromatography. As shown in Figure 2, starting from a molar ratio of enzyme : PEG of 1:1, no free GlucDH2 was observed; increasing the molar ratio to 1:1.5, the proportion of large cross-linking products increased.

The cross-linked enzyme produced in Example 2 was included into a first layer of a two-layer glucose test strip and was evaluated regarding stability and mobility of the enzyme. As shown in Table 2, an increase of the molar ratio of enzyme : PEG leads to the production of larger cross-linking products. The molecular weight of GlucDH2 thus produced, as determined by gel permeation chromatography, increases from 112 kDa (free enzyme) up to 2,000 kDa when a molar ratio of 1:1.5 is used. Correspondingly, the mobility of the enzyme in the test strip strongly decreases with increasing size of the cross-link product. Stability in the test strip was tested at 35°C and 85 % relative humidity and was not significantly different as compared to free GlucDH2.

### Example 3:

In further experiments, NHS-activated PEGs with molecular masses > 3,000 g/mol were tested; however, it was found that cross-linking was less efficient than in Example 2. Moreover, cross-linking products obtained with NHS-activated PEG with molecular masses of 10,000 and 20,000 g/mol resulted in very viscous product,s which made further processing difficult. Accordingly, PEGs with molecular masses of > 3,000 g/mol were found to be less preferred for cross-linking GlucDH2.

### Example 4:

For the following experiments, the following standard reaction conditions were used for cross-linking GlucDH2: To a solution of 2.85 mM GlucDH2 (80 mg/ml protein solution) in 0.2 molar sodium bicarbonate buffer pH 8.3, NHS-PEG in DMSO was added to a concentration of 4.2 mM while stirring at room temperature by slow dropping. The resulting viscous solution was dialyzed 3 times against 0.05 molar sodium/potassium phosphate buffer pH 7.0 comprising 0.2 molar NaCL and once against 0.05 molar potassium/sodium phosphate buffer pH 7.0. The cross-linking products thus produced were further characterized.

### a) Immobilization Test (Supernatant Test)

As a first test for immobilization of the enzyme, 20 µl buffer were pipetted onto a test chemistry comprising the respective GlucDH2 preparation and were incubated for 1 minute. After this time, 10 µl of the buffer were withdrawn and analyzed for GlucDH2 activity. The activity diffusing into the buffer is expressed in Table 2 as a percentage of total activity applied. As can be seen from Table 2, the amount of diffusible enzyme decreases drastically with increasing molecular weight of the cross-link products.

### b) Diffusion Test (Fluorescence Microscopy)

For this assay, GlucDH2 was chemically coupled to the fluorescent dye Alexa647, which can be excited at a wavelength of 650 nm, leading to a fluorescence emission at 668 nm. Fluorescently labeled enzyme was used in the cross-linking reaction as described above and was applied to a first layer of 2-layered glucose test strips. The strips were stored under various conditions for various periods of time, whereafter localization of the enzyme was examined by fluorescence microscopy (Figures 4 and 5). It was found that diffusion of the enzyme into the second layer could essentially be prevented.

### Example 5: Stability of the cross-linked GlucDH2

Test strips produced according to the method and stored under the conditions indicated in Example 4 were also examined for remaining activity after several days (Figure 6). It was found that under dry and warm conditions (35 °C / 0% relative humidity), no significant difference between free enzyme and cross-linked enzyme was found. In contrast, cross-linked enzyme showed a 20 % higher residual activity after 55 days under moist and warm conditions (35 °C / 85% relative humidity).

For the aforesaid test strips, the supernatant test (Example 4a)) for mobility was also performed. It was found that free GlucDH2 had an average mobility of 3 to 6%, whereas cross-linked GlucDH2 had a mobility of less than 1 % under all conditions, indicating immobilization of the enzyme in the first layer of the test strip (Fig. 7).

### Example 6:

To compare kinetics and signal dynamics of free GlucDH2 and cross-linked GlucDH2, test strips comprising either of said enzyme preparations were tested for kinetic and signal dynamic parameters. It was found that cross-linking the enzyme does not result in a significant difference. Surprisingly, in colorimetric test strips using cNAD chemistry, the diffusion of cNADH apparently is so fast that an increased production of color in the first layer, which is the layer examined photometrically, cannot be observed. In contrast, a different chemistry producing heteropolyblue as a final colored reaction product shows a different behavior: In particular at glucose concentrations of less than 200 mg/dl, glucose dependence of the resulting signal is much stronger if cross-linked enzyme is used as compared to free enzyme, leading to a significantly improved performance of the system.

### List of reference numbers

- 120: glucose test strip
- 122: buffer
- 124: pipet
- 128: first layer of glucose test strip
- 130: second layer of glucose test strip

## Claims

1. A method for cross-linking polypeptide molecules, comprising the step of combining a cross-linking agent and polypeptide molecules in solution under conditions suitable for a cross-linking reaction to occur.

2. The method of claim 1, wherein the polypeptide is an enzyme.

3. The method of claim 1 or 2 wherein the cross-linking agent is an unbranched molecule chemically activated at both ends.

4. The method of any one of claims 1 to 3, wherein the cross-linking agent is bis-activated polyethylene glycol.

5. The method of any one of claims 1 to 4, wherein the cross-linking agent has a molecular mass of 0.6 kDa to 20 kDa, preferably 0.9 kDa to 10 kDa, more preferably 1 kDa to 5 kDa, most preferably 3 kDa.

6. The method of any one of claims 1 to 5, wherein the molar ratio of crosslinking agent / polypeptide molecules is 0.2 to 5, preferably 0.5 to 4, more preferably 0.8 to 3, most preferably 1 to 2.

7. The method of any one of claims 1 to 6, wherein at least a fraction of the polypeptide molecules are glucose dehydrogenase molecules, preferably wherein at least 80% of the polypeptide molecules are glucose dehydrogenase molecules, more preferably wherein at least 90% of the polypeptide molecules are glucose dehydrogenase molecules.

8. A preparation comprising cross-linked polypeptide molecules, obtainable by the method of any one of claims 1 to 7.

9. Cross-linked polypeptide molecules, wherein said polypeptide molecules are cross-linked by essentially unbranched cross-linking groups comprising at least 40 contiguous atoms.

10. The cross-linked polypeptide molecules of claim 9, wherein the polypeptide is an enzyme, preferably a dehydrogenase.

11. A test chemistry matrix comprising a redox cofactor, an agent capable of eliciting a change in at least one measurable property of an indicator reagent in the presence of redox equivalents, and an indicator reagent, wherein said chemistry matrix further comprises a preparation of cross-linked polypeptide molecules obtainable by the method according to any one of claims 1 to 7 and/or cross-linked polypeptide molecules according to claim 9 or 10.

12. A diagnostic test element for the determination of an analyte from a body fluid sample comprising the test chemistry matrix of claim 11 on a carrier.

13. A preparation of cross-linked polypeptide molecules obtainable by the method according to any one of claims 1 to 7 and/or cross-linked polypeptide molecules according to claim 9 or 10 for use in the diagnosis of disease.

14. A preparation of cross-linked polypeptide molecules obtainable by the method according to any one of claims 1 to 7 and/or cross-linked polypeptide molecules according to claim 9 or 10 for use in the diagnosis of diabetes.

15. A method for producing a test chemistry matrix, comprising the steps of
a) obtaining a preparation of cross-linked enzyme molecules according to the method of any one of claims 2 to 7 and/or cross-linked enzyme molecules according to claim 10, and
b) admixing a redox cofactor, an agent capable of eliciting a change in at least one measurable property of an indicator reagent in the presence of redox equivalents, an indicator reagent, and the preparation of cross-linked enzyme molecules obtained in step a), and, thereby,
c) producing a test chemistry matrix.
